# EUROPÄISCHE PATENTSCHRIFT

(11) **EP 0 790 114 B1**
(45) Veröffentlichungstag und Bekanntmachung des Hinweises auf die Patenterteilung: **07.06.2000**
(21) Anmeldenummer: 96810867.0
(22) Anmeldetag: 12.12.1996
(51) Int. Cl.: B29C 41/04, B29C 33/34, B29C 41/42, B29C 41/46, B29L 23/00

(54) **Anlage zur Herstellung von Kunststoffrohren im Schleudergussverfahren**
Apparatus for producing plastic pipes by rotational moulding
Installation pour la fabrication de tuyaux en matière plastique moulés par rotation

(30) Priorität: 22.12.1995 CH 366595
(43) Veröffentlichungstag der Anmeldung: 20.08.1997
(73) Patentinhaber: Hobas Engineering AG, CH-4054 Basel (CH)
(72) Erfinder: Carlström, Bengt, 9220 Felden (AT)
(74) Vertreter: Eder, Carl E.

(56) Entgegenhaltungen:
- WO-A-93/08009
- CH-A- 684 326
- DE-A- 2 911 369
- DE-A- 3 110 091
- FR-A- 1 134 889
- FR-A- 2 612 831
- US-A- 3 541 645

## Beschreibung

Die vorliegende Erfindung betrifft eine Anlage zur Herstellung von Kunststoffrohren im Schleudergussverfahren aus folgenden Ausgangsmaterialien: Füllstoff enthaltendem Kunstharz, Glasfasern und gewünschtenfalls weiteren Zuschlagstoffen wie z.B. Sand. Derartige Anlagen sind unter anderem aus der schweizerischen Patentschrift 684 326 und der PCT WO 93/08009 bekannt. Diese bekannten Anlagen dienen dazu, Rohre grosser Länge, also beispielsweise von 6 und mehr Metern, und einem Durchmesser von mindestens 30 cm, herzustellen. Es hat sich nun gezeigt, dass die Herstellung von Rohren mit einem kleineren Durchmesser, also einen Durchmesser von beispielsweise 10 - 20 cm, mittels solcher Anlagen allenfalls möglich ist, dass jedoch der Wunsch besteht, für die Herstellung solcher Rohre Anlagen zu haben, die eine grössere Kapazität aufweisen, ohne dass jedoch der dafür nötige Raumbedarf proportional zur Leistung zunimmt.

Aus der deutschen Offenlegungsschrift 29 11 369 ist eine Anlage zur Herstellung von Rohren aus Polyamid bekannt. Bei dieser Anlage wird Caprolactam oder Laurinlactam mit einer bestimmten Ausgangstemperatur in eine Drehform eingebracht. Dann wird zur Bildung eines Rohres das eingebrachte Material durch rasches Drehen der Drehform an die Wandung der Form geschleudert. Nach dem Schleudervorgang lässt man die Drehform während einer Zeit, die das Mehrfache der Schleuderzeit beträgt, abkühlen, damit der Durchmesser des entstandenen Rohres sich etwas reduziert und das Rohr leicht aus der Drehform genommen werden kann.

Eine der beschriebenen Anlagen weist ein Trommelmagazin mit fünf in diesem angeordneten beidseits verschiebbaren und beheizbaren zylindrischen Kammern auf, wobei in jeder dieser Kammern eine achsial aus ihr herausnehmbare Drehform untergebracht ist. Zur Erzeugung der Schleuderbewegung der Drehform dient ein einziger Antriebsmotor. Durch schrittweises Drehen des Trommelmagazines kann eine Kammer nach der andern in die Stellung gebracht werden, in welcher die in ihr untergebrachte Drehform durch den Antriebsmotor in Rotation gebracht werden kann. Nach mehreren Drehschritten des Trommelmagazins, während denen sich die Temperatur des Produktes erniedrigt, wird die Drehform mit dem Fertigprodukt axial aus der Kammer herausgefahren und dann wird das entstandene Rohr aus der Drehform herausgezogen.

Der Nachteil dieser Anlage besteht darin, dass sich mit ihr nur solche Rohre herstellen lassen, die aus homogenem Material bestehen, also weder Rohre, deren Wandung eingelagerte Bestandteile aufweisen, noch solche, deren Wandung aus unterschiedlichen aber festmiteinander verbundenen Schichten gebildet ist.

Mit der vorliegenden Erfindung wird nun die Aufgabe gelöst, eine Maschine oder Anlage zur Verfügung zu stellen, mit welcher sich verhältnismässig dünne Rohre, also Rohre mit einem Durchmesser von 10 - 50 cm, und mit einem frei wählbaren Schichtaufbau der Wandung mit einem Aufwand herstellen lassen, der dem auf das Gewicht bezogenenen Aufwand der Rohre mit grösserem Durchmesser entspricht. Dies wird dadurch erreicht, dass man die Anlage derart ausgestaltet, dass sich mit ihr mehrere Rohre auf verhältnismässig engem Raum gleichzeitig so herstellen lassen, dass während der Herstellung des einen Rohres zeitlich gestaffelt mit der Herstellung von mindestens fünf weiteren Rohren begonnen wird. Eine derartige Maschine oder Anlage nach dem Oberbegriff des Anspruchs 1 ist nun dadurch gekennzeichnet,
- dass das Drehgestell sechs bis acht Schleudergussmatrizen aufweist, von denen jede mit einem Antriebsmotor versehen ist,
- dass zum schrittweisen Drehen des Drehgestells eine Antriebs- und Fixiervorrichtung vorhanden ist,
- dass eine in Betrieb nur achsparallel verschiebbare Eintragvorrichtung vorhanden ist, um die Materialien, aus denen das Rohr aufgebaut wird, in eine oder gleichzeitig in zwei Matrizen einzubringen,
- dass eine Ausformvorrichtung vorhanden ist, um an einer ortsfesten Stelle je ein fertiges Rohr aus einer Matrize herauszuziehen,
- dass eine Steuervorrichtung vorhanden ist, die folgendes steuert:
   = das schrittweise Drehen des Drehgestells,
   = das Rotieren jeder Matrize mit einer von der Position, in der sie sich befindet, abhängigen Tourenzahl,
   = die Verschiebebewegung der Eintragvorrichtung relativ zum Drehgestell, sowie die momentane und die totale Menge der einzubringenden Materialien und
   = die Funktionsweise der Ausformvorrichtung.

Nachfolgend wird anhand der beiliegenden, ziemlich stark schematisierten Zeichnung ein Ausführungsbeispiel der Erfindung beschrieben. in der Zeichnung, in welcher die einzelnen Figuren in unterschiedlichen Massstäben dargestellt sind, zeigt
die Fig. 1 eine Übersicht über die ganze Anlage, jedoch ohne Heizelemente, ohne Absaugvorrichtung, ohne Ausformvorrichtung und ohne die Steuervorrichtung,
die Fig. 2 Details der Antriebsvorrichtung für das Drehgestell,
die Fig. 3 die Antriebs- und Fixiervorrichtung des Drehgestells,
die Fig. 4 eine Stirnansicht auf das Drehgestell,
die Fig. 5 eine perspektivische Ansicht einer einzelnen Schleudergussmatrize,
die Fig. 6 und 7 je eine perspektivische Darstellung der Abschlussringscheibe,
die Fig. 8 einen Schnitt durch das eine Ende einer Schleudergussmatrize und
die Fig. 9 und 10 den wesentlichen Teil der Ausziehvorrichtung in zwei verschiedenen Betriebszuständen.

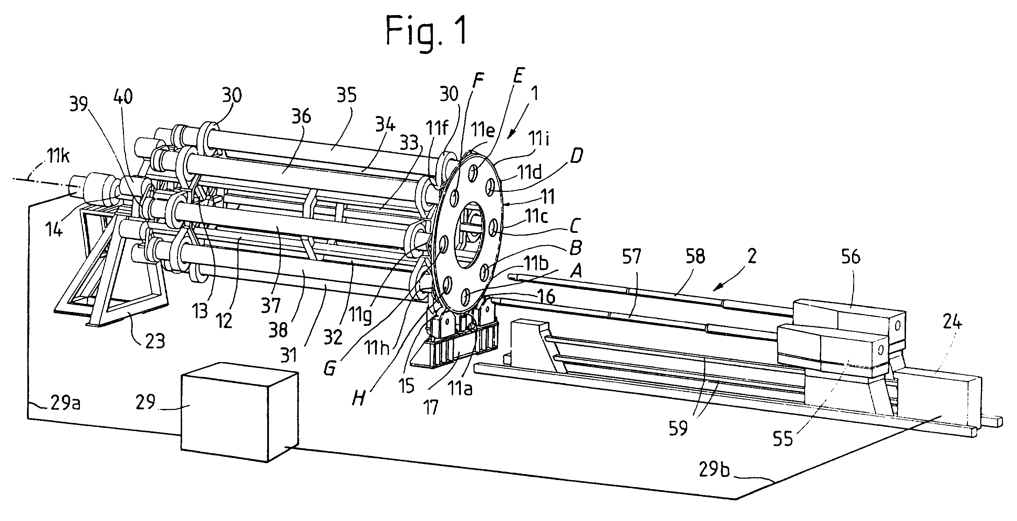

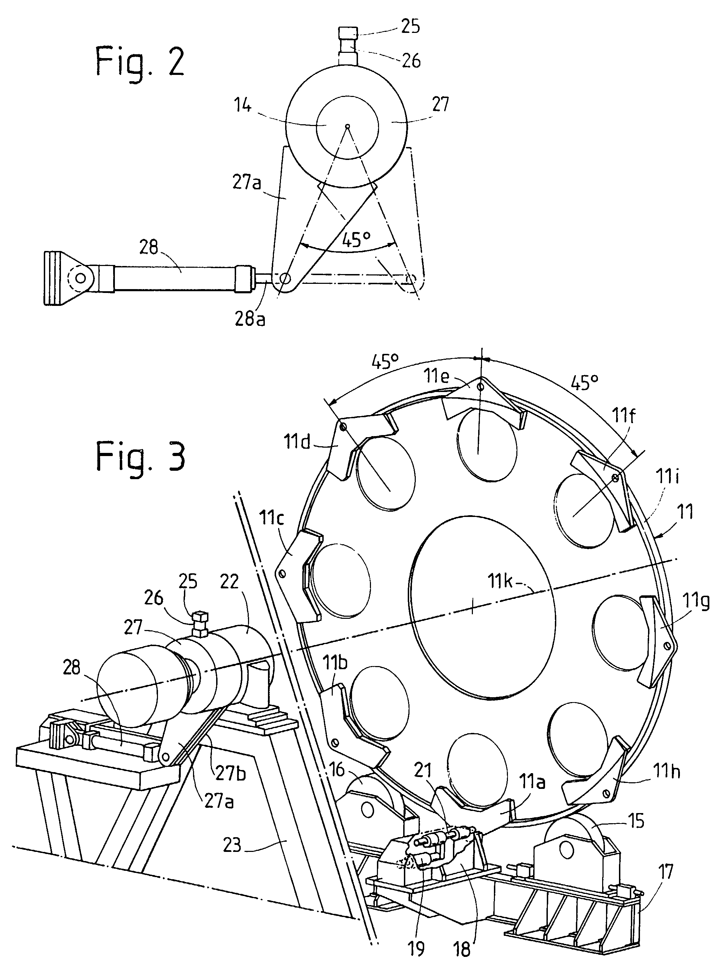

Aus der in der Fig. 1 sehr schematisch dargestellten Übersicht über die ganze Anlage ist ersichtlich, dass sie im wesentlichen aus zwei Teilen besteht, einem horizontalachsigen, hier als Ganzes mit 1 bezeichneten Drehgestell und einer als Ganzes mit 2 bezeichneten Eintragvorrichtung. Das Drehgestell 1 weist an dem der Eintragvorrichtung 2 zugewandten Ende eine Kreisscheibe 11 auf, die über mehrere Stangen 12 und Speichen 13 mit einem zur Kreisscheibe 11 koaxialen Wellenstummel 14 verbunden ist. Die mit einem Reifen 11i versehene Kreisscheibe selber ist, wie man aus der Fig. 3 ersehen kann, auf zwei frei drehbaren Rollen 15 und 16 gelagert, die ihrerseits auf einer Traverse 17 frei drehbar gehalten werden.

An der Kreisscheibe 11 sind acht regelmässig verteilt angeordnete Fixierlappen 11a, 11b, 11c, 11d, 11e, 11f, 11g und 11h befestigt, von denen jeder mit einem Loch versehen ist. In einem an der Traverse 17 befestigten Gehäuse 18 ist ein durch einen Elektromagneten 19 betätigbarer Fixierstift 21 untergebracht, der sich in das Loch des in bezug auf die Scheibenachse 11k, die auch die Drehgestellachse ist, unten befindlichen Fixierlappens, also in der Zeichnung in das Loch des Fixierlappens 11a, einschieben lässt, um die Kreisscheibe 11 gegen Drehung zu sichern. Am andern Ende des Drehgestells 1 ist der zur Kreisscheibe 11 koaxiale Wellenstummel 14 in einem Lager 22 frei drehbar gelagert, welches seinerseits in einem Gestell 23 festgehalten wird. Auf dem freien Ende dieses Wellenstummels ist die mit einem durch einen Elektromagneten 25 betätigbaren Mitnehmerstift 26 versehene Mitnehmerhülse 27 gelagert. Im Bereich dieses Mitnehmerstiftes 26 weist der Wellenstummel 14 acht regelmässig verteilt angeordnete Löcher, Rillen oder Nuten auf, damit der Mitnehmerstift 26 mit der Welle in Eingriff kommen kann. Die Mitnehmerhülse 27 ihrerseits ist mit zwei Armen 27a und 27b versehen, deren freie Enden an den Kolben 28a eines pneumatischen oder hydraulischen Zylinders 28 angelekt sind.

Dabei sind die Abmessungen der Arme 27a, 27b und der Verschiebeweg des Kolbens 28a so bemessen, dass bei einer Kolbenbewegung die Mitnehmerhülse 27 eine Verschwenkung von 45° erfährt.

Dies hat zur Folge, dass das Drehgestell nach acht Drehschritten wieder seine ursprüngliche Lage eingenommen hat. Es sind daher die Positionen oder Stellen, an denen sich die einzelnen Schleudergussmatrizen bei ruhendem Drehgestell 1 befinden mit A, B, C, D, E, F, G und H bezeichnet, wobei die mit A bezeichnete Position die unterste Position ist und die übrigen sich im Gegenuhrzeigersinn folgen, wenn man von der Eintragvorrichtung 2 gegen das Drehgestell blickt.

An den Stangen 12 des Drehgestells 1 sind sechzehn Lager 30, von denen je zwei für die Lagerung einer der acht Schleudergussmatrizen 31, 32, 33, 34, 35, 36, 37 und 38 dienen, derart befestigt, dass sie diese Matrizen achsparallel zur Drehgestellachse 11k halten. Jede dieser acht Matrizen ist über einen Riemenantrieb 39 mit einem ihr zugeordneten Antriebsmotor 40 verbunden.

Obwohl in der Zeichnung alle acht Schleudergussmatrizen den gleichen Innendurchmesser aufweisen, ist es ohne weiteres möglich, auf einem Drehgestell Schleudergussmatrizen mit unterschiedlichen Innendurchmessern anzuordnen. In einem solchen Fall wird es zweckmässig sein, jede Schleudergussmatrize mit zwei Lagern 30 zu versehen und die Lagergehäuse 30a so auszugestalten, dass sie sich mit einfachen Mitteln auf dem Drehgestell 1 befestigen und von ihm wieder lösen lassen, wobei die Lagergehäuse 30a so zu dimensionieren sind, dass, unabhängig vom Innendurchmesser der einzelnen Matrizen, die geometrische Achse 31a aller auf dem Drehgestell 1 angebrachten Matrizen den gleichen Abstand zur Drehgestellachse 11k besitzen. In einem solchen Fall ist es jedoch von Vorteil, wenn die Riemenscheiben 31b aller Matrizen den gleichen Aussendurchmesser haben.

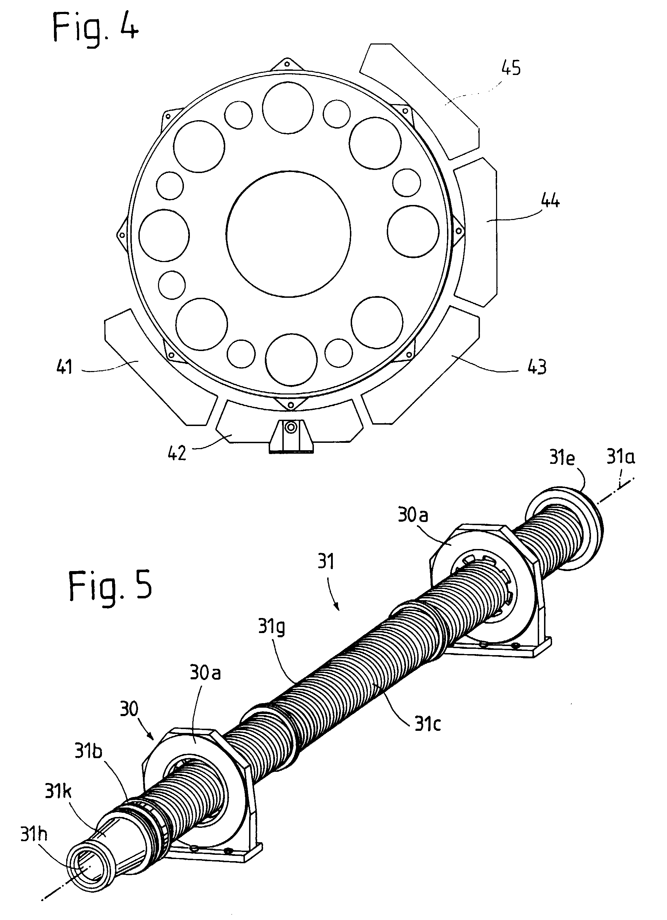

Wie aus der Fig. 4 ersichtlich ist, sind ausserhalb des Drehgestells 1 fünf ortsfeste Heizkörper 41, 42, 43, 44 und 45 angeordnet, die das Gestell 1 teilweise umgeben. Jeder Heizkörper erstreckt sich über die ganze Länge der Schleudergussmatrizen, so dass er ohne weiteres die sich unmittelbar in seiner Nachbarschaft befindliche Schleudergussmatrize auf eine vorbestimmte Temperatur aufheizen bzw. die aufgeheizte Matrize auf einer bestimmten Temperatur halten kann. In der gezeichneten Stellung des Drehgestells 1 können also die Schleudergussmatrizen 38, 31, 32, 33 und 34 aufgeheizt werden, also die Matrizen, die sich in den Positionen H, A, B, C und D befinden. Jeder Heizkörper enthält ein oder mehrere Heizelemente, die elektrisch oder mit heisser Luft oder mit Heisswasser oder mit Dampf beheizt werden können, wobei mittels der nachfolgend noch beschriebenen Steuervorrichtung 20 die für die Rohrherstellung nötige Temperatur gesteuert wird.

Die Beheizung der Matrizen kann jedoch auch auf andere Art und Weise erfolgen: Die in der Fig. 5 dargestellte Matrize 31 besitzt ein auf ihr aufgewickeltes elektrisches Heizelement 31c, dessen Zuleitung über eine nicht dargestellte Schleifringverbindung mit einer durch die Steuervorrichtung steuerbaren Stromquelle verbunden ist.

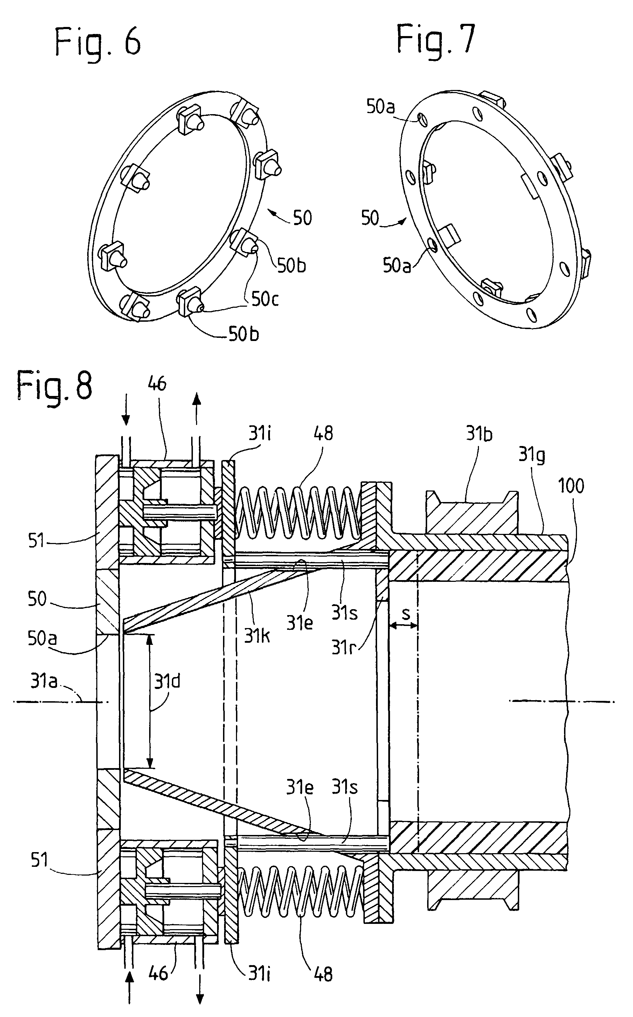

Wie aus den Fig. 5 und 8 ersichtlich ist, weist jede Schleudergussmatrize 31 an ihrem der Eintrags-Öffnung 31e abgewandten Ende ein konisches Endstück 31k auf, wobei zweckmässigerweise alle Endstücke unabhängig vom Innendurchmesser des Rohres den gleichen Öffnungsdurchmesser 31d besitzen. Diese mit 31f bezeichnten Öffnungen liegen an der in den Fig. 6 und 7 dargestellten stationären Abschlussringscheibe 50 an, die mit acht hinter je einer Eintrittsöffnung 50a angeordneten Absaug-Ventilatoren 50b versehen ist, deren Ausgangsöffnungen 50c in einen nicht gezeichneten Ausblaskanal münden.

Jede Schleudergussmatrize weist, wie man besonders gut aus der Fig. 8 ersehen kann, vor dem Beginn der Konusfläche des konischen Endstücks 31k einen ringförmigen Einsatz 31r auf, der einerseits dazu dient, dem entstehenden Schleudergussrohr 100 eine genau definierte Stirnfläche zu geben und andererseits den vier Ausstossstangen 31s als Führung dient. Diese vier Stangen sind je durch einen Schlitz 31e des konischen Endstücks 31k durch dieses hindurch nach aussen geführt, wo sie auf einen Ring 31i befestigt sind.

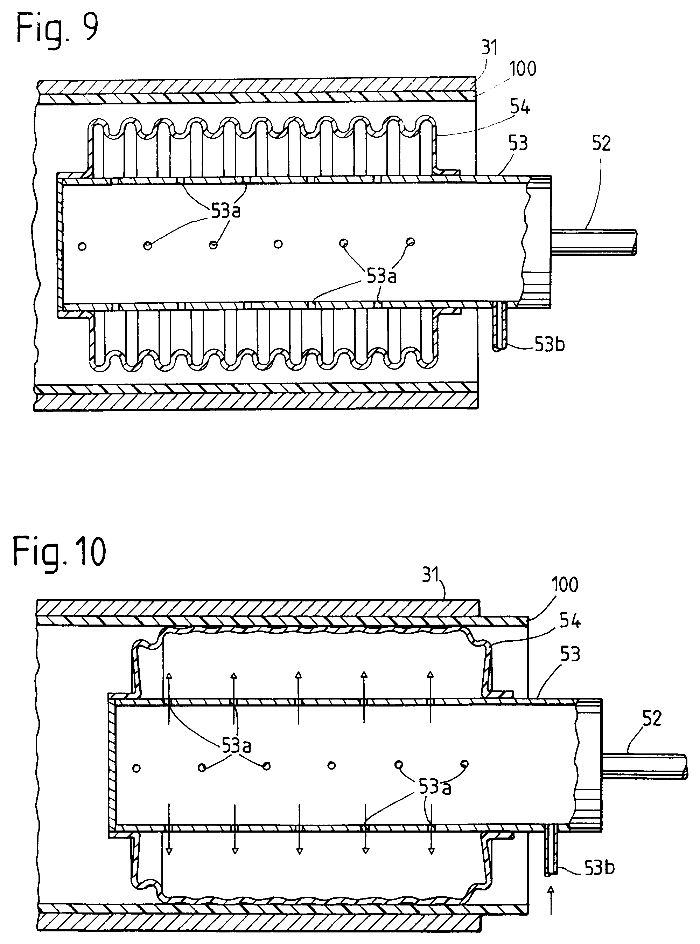

An der Abschlussringscheibe 50 sind an der Position G zwei sich radial erstreckende Lappen 51 angebracht, an welchen je ein Ausstoss-Zylinder 46 befestigt ist, dessen Kolbenstange dazu dient, den Ring 31i entgegen der Kraft von zwei Rückstellfedern 48 von der Abschlussringscheibe 50 wegzustossen und damit das fertige Schleudergussrohr 100 um die Strecke s, d. h. einige wenige cm nach rechts, also in der Richtung zur Eintragöffnung 31e zu verschieben, damit es nicht mehr an der Innenwand der Schleudergussmatrize 31 klebt und sich mühelos mittels einer Ausziehvorrichtung aus ihr herausziehen lässt. Zu diesem Zweck ist an der andern Seite der Schleudergussmatrize 31 an der gleichen Position G die Auszugvorrichtung angeordnet, deren wesentliche Bestandteile in den Fig. 9 und 10 dargestellt sind. Sie besitzt eine in ihrer Längsrichtung verschiebbare Auszugsstange 52. An ihrem vorderen, der Schleudergussmatrize 31 zugewandten freien Ende ist ein Zylinder 53 angebracht mit einem Anschluss 53b für die Zufuhr von Druckluft und das Absaugen von Luft. Auf diesem mit mehreren radialen Bohrungen 53a sitzt ein geschlossener Gummibalg 54, der sich durch die aus den Bohrungen 53a in ihn hinein strömende Druckluft so aufblasen lässt, dass er auf seiner ganzen Länge an der Innenfläche eines in der Schleudergussmatrize 31 hergestellten Kunststoffrohres 100 satt und fest anliegen kann, wenn sich der Zylinder 53 innerhalb eines solchen Kunststoffrohres befindet.

Die als Ganzes mit 2 bezeichnete Eintragvorrichtung weist zwei miteinander verbundene Sockel 55 und 56 auf, die an einer zur Drehgestellachse 11k parallelen Schienenführung 59 verschiebbar geführt sind und sich mittels eines im Gehäuse 24 untergebrachten Motors über nicht sichtbare Seilzüge aus der gezeichneten Stellung nach links und wieder zurück in die gezeichnete Stellung verschiebbar sind. Jeder dieser beiden Sockel 55 und 56 enthält, wie das aus der PCT/WO93/08009 bekannt ist, mit Dosierpumpen versehene Vorratsbehälter für die einzelnen Komponenten des flüssigen Kunststoffes, d. h. des Harzes und des Härters, sowie für den Sand und auch den nötigen Speicherplatz für die Glasfaserschnurspulen. Jeder der beiden Sockel 55 und 56 ist mit einem Beschickungsarm 57 bzw. 58 versehen, in welchem die Transport- und die Ausgabevorrichtungen für diese Ausgangsmaterialien untergebracht sind, unter anderem also die an deren Enden mit Düsen versehenen Rohren für die flüssigen Kunststoff-Komponenten, eine Förderschnecke für den Sandtransport sowie für die Glasfaserschnüre eine Zugvorrichtung und rotierende Schneidemesser. Die geometrische Anordnung ist dabei so ausgestaltet, dass der Beschickungsarm 57 in die Eintragsöffnung 31e einer sich an der Position A befindlichen Schleudergussmatrize und der Beschickungsarm 58 gleichzeitig in die Eintragsöffnung der benachbarten, sich an der Postion B befindlichen Matrize 32 eingeführt wird. Die beiden Beschickungsarme 57 und 58 sind so lang, dass sich ihr freies Ende, an welchem die Ausgabeöffnungen für das Kunststoffharz, den Sand und die Glasfasern angeordnet sind, am Ende des Verschiebungsweges an der Stelle innerhalb einer Schleudergussmatrize befindet, an welcher deren zylindrischer Abschnitt 31g an den konischen Abschnitt 31k angeschlossen ist.

In einem Kasten 29, der durch Leitungen 29a mit dem Drehgestell 1 und durch Leitungen 29b mit der Eintragvorrichtung 2 verbunden ist, sind die mit Rechnern verbundenen Steuerungs- sowie die Mess- und Uberwachungsvorrichtungen für die ganze Anlage untergebracht, unter anderem also für das Drehgestell 1 die Steuergeräte für die aus den Teilen 22, 25, 26, 27 und 28 bestehende Antriebsvorrichtung des Drehgestells, für die aus den Teilen 19 und 21 bestehende Fixiervorrichtung, für die Antriebsmotoren 40, für die Absaugventilatoren 50b, für die druckluftbetätigten Zylinder 46 der Ausstossvorrichtung, für die Betätigung der Auszugsvorrichtung, nämlich zum Verschieben der Auszugsstange 52 und zum Aufblasen und Evakuieren des Gummibalges 54, sowie für die durch Programme und an den Matrizen angebrachte Temperaturfühler gesteuerten Schalter zum Ein- und Ausschalten der Matrizenheizungen.

Selbstverständlich kann auch noch eine in der Zeichnung nicht dargestellte Vorrichtung vorhanden sein, mit der sich in jede Schleudergussmatrize nach dem Entfernen eines fertig hergestellten Rohres, aber vor dem Eintrag der zur Herstellung des nächsten Rohres dienenden Materialien ein Trennmittel einbringen lässt, um das Festkleben des Materials an der Matrizenwand zu verhindern.

Zu den Antriebs- und Steuervorrichtungen für die Eintragvorrichtung gehören unter anderem die Steuerorgane für das Hin- und Herschieben dieser Vorrichtung sowie natürlich die Mess- und Dosiervorrichtungen für die Ausgabe der Baustoffe zur Herstellung der Rohre, also der Dosierpumpen für die Harzbestandteile, der Förderschnecke für den Sand sowie der Förder- und Zerkleinerungsvorrichtung für die Glasfasern.

Nachfolgend wird nun noch die Arbeitsweise der vorgehend beschriebenen Anlage erklärt.

Im dargestellten Zustand ist das Drehgestell 1 durch den im Fixierlappen 11a steckenden Fixierstift 21 gegen Verdrehen gesichert. Es befindet sich die Matrize 31 an der Position A, die Matrize 32 an der Position B, die Matrize 33 an der Position C, die Matrize 34 an der Position D, die Matrize 35 an der Position E, die Matrize 36 an der Position F, die Matrize 37 an der Position G und die Matrize 38 an der Position H. Alle Matrizen mit Ausnahme der Matrize 37 an der Position G werden durch ihren zugehörigen Motor 40 über den Riemenantrieb 39 angetrieben, beispielsweise so, dass die Tourenzahl an der Position H 200 min.⁻¹, an der Position A 455 min.⁻¹, an der Position B 655 min.⁻¹, an den Positionen C, H, D und E ca. 300 min.⁻¹ beträgt und an der Position F die Tourenzahl abnimmt, bis die Matrize still steht.

In die an der Position H befindliche Matrize 38 wird mittels einer in der Zeichnung nicht sichtbaren Düse ein Trennmittel angespritzt, das verhindert, dass die nachfolgend eingebrachten, zum Aufbau eines Rohres dienenden Baustoffe an der Matrizenwand festkleben. Die Matrize dreht sich dabei mit einer Tourenzahl, die die gleichmässige Verteilung des Trennmittels auf die ganze Innenfläche gewährleistet.

An den Positionen A und B fahren gleichzeitig Beschickungsarme 57 bzw. 58 mit konstanter Verschiebegeschwindigkeit in die vor ihnen stehenden und sich mit grosser Tourenzahl drehenden Matrizen 31 bzw. 32 ein und aus diesen wieder heraus. Dies kann ein oder mehrmals geschehen. Dabei geben sie die für den Aufbau eines glasfaserarmierten Kunststoffrohres 100 nötigen Baustoffe, also mit Füllstoffen angereichertes Harz und Härter, sowie Sand und Glasfasern nach einem im Steuergerät gespeicherten Programm ab, wobei der Beschickungsarm 57 die äusseren Schichten des Rohres erzeugt, der Beschickungsarm 58 aber die inneren Schichten. Da die Matrizenheizung in Betrieb ist, beginnt gleichzeitig die Aushärtung des Kunststoffharzes.

An den Positionen C, D und E härtet das eingebrachte Material, aus welchem durch das Schleudern ein Rohr entstanden ist, bei etwas reduzierter Tourenzahl vollständig aus, wobei zu Beginn, also an der Position C, wo das Material noch ziemlich weich ist, auch noch eine Verdichtung erfolgen kann. Je nach Bedarf, der durch an den Matrizen angebrachte Thermofühler festgestellt wird und der vom Rohrdurchmesser, die Rohrwandstärke und natürlich auch von der vorhandenen Raumtemperatur abhängig ist, lässt sich an den Positionen H, A, B, C und D durch die Heizelemente Wärme zuführen. Das so erzeugte Rohr härtet nun an der Position F noch fertig aus, wobei die Tourenzahl der Matrize verlangsamt wird, bis die Matrize still steht. Zu gleicher Zeit wird in der sich in der Position G befindlichen Matrize zuerst der Ausstosszylinder 46 betätigt, um das allenfalls satt in der Matrize sitzende Kunststoffrohr 100 von ihr zu lösen, wie das weiter vorn beschrieben ist. Gleichzeitig fährt von der andern Seite die Auszugvorrichtung soweit in das Kunststoffrohr 100 ein, dass sich sein Gummi-Balg 54 vollständig innerhalb des Rohres befindet. Sobald dieses etwas ausgestossen ist, wird der Gummibalg durch einströmende Druckluft so stark aufgeblasen, dass er fest im Rohr sitzt und dieses beim anschliessenden Verschieben der Auszugstange 52 nach rechts herausziehen kann.

An allen den Stellen, an denen beim Aushärten des Kunststoffes Dämpfe oder Gase frei werden, wird durch die Steuervorrichtung der entsprechende Ventilator 50b eingeschaltet, der diese Gase in den Abgaskanal bläst.

Wenn nun alle diese vorstehend beschriebenen, gleichzeitig ablaufenden Bewegungen beendigt sind, zieht der Elektromagnet 19 den Fixierstift 21 aus dem Loch des Fixierlappens 11a, worauf der Kolben 28a des Hydraulikzylinders 28 ausgefahren wird und den Wellenstummel 14 und damit das ganze Drehgestell 1, dessen Kreisscheibe 11 auf den beiden Rollen 15 und 16 abrollt, um 45° verdreht, so dass der Fixierlappen 11b vor dem Fixierstift 21 steht, welcher durch den Elektromagneten 21 in das Loch dieses Filterlappens geschoben wird, wodurch das Gestell in der neuen Lage wieder fixiert wird.

In dieser neuen Lage befindet sich also die Matrize 31 an der Position B, die Matrize 32 an der Position C, usw., d. h., in jeder Matrize findet ein nächster Arbeitsprozess statt, wie diese vorstehend beschrieben sind. Es wird also nach jeder Achtels-Drehung ein fertiges Kunststoffrohr ausgezogen. Falls, wie das ohne weiteres möglich ist, für das Entriegeln und Verriegeln eines Fixierlappens 2,5 s und für das Drehen des Gestells 1 um 45° 7 s benötigt werden, so stehen bei einem 192-Sekunden-Takt 180 s für das mehrmalige Ein- und Ausfahren, jedes der beiden Beschickungsarme 57 und 58 zur Verfügung, also für das Einbringen des zum Aufbau eines Rohres nötigen Materials insgesamt 6 min., was erfahrungsgemäss im allgemeinen völlig ausreichend ist. Mit anderen Worten, es wird zur Herstellung eines Rohres eine Zeit von 25 min. 36 s benötigt, die Anlage liefert also jede 192 Sekunden ein Rohr oder in 4 Stunden 75 Rohre à beispielsweise 6 m, also 450 Laufmeterrohre.

Mit einer derartigen Produktionsleistung sind Herstellungskosten solcher qualitativ hochstehender, armierter Kunststoffrohre nicht grösser als die Herstellungskosten metallener Rohre. Da solche Kunststoffrohre aber wesentlich leichter sind, sind die Transport- und Verlegekosten geringer als bei Metallrohren. Da zudem die Lebensdauer ein Vielfaches derjenigen von Metallrohren ist, bieten derart hergestellte Rohre einen wesentlichen Fortschritt gegenüber allen bisher bekannten Rohren vergleichbarer Abmessungen.

## Patentansprüche

1. Zur Herstellung von aus Füllstoff enthaltendem Kunststoff, Glasfasern und gewünschtenfalls weiteren Zuschlagstoffen bestehenden Rohren im Schleudergussverfahren dienende Anlage mit einem horizontalachsigen Drehgestell (1), auf welchem mehrere horizontalachsig drehbar gelagerte, motorisch antreibbare Schleudergussmatrizen (31, 32, 33, 34, 35, 36, 37, 38) in gleichem Achs-Abstand von der Drehgestell-Achse (llk) gleichmässig verteilt angeordnet sind, wobei das Drehgestell (1) derart schrittweise drehbar ist, dass bei jedem Drehschritt eine Matrize (32) in die Position gelangt in welcher sich vorher die benachbarte Matrize (31) befand, wobei Heizelemente (41, 42, 43, 44, 45; 31c) vorhanden sind, um jede Matrize (31, 32, 33, 34, 35, 36, 37, 38) in wenigstens einer ihrer Positionen zu beheizen, und wobei eine Absaugvorrichtung (50b) vorhanden ist, um die beim Aushärten frei werdenden Gase aus den einzelnen Matrizen abzusaugen, dadurch gekennzeichnet,
- dass das Drehgestell sechs bis acht Schleudergussmatrizen (31, 32, 33, 34, 35, 36, 37, 38) aufweist, von denen jede mit einem Antriebsmotor (40) versehen ist,
- dass zum schrittweisen Drehen des Drehgestells (1) eine Antriebs- (25, 26, 27, 28) und Fixiervorrichtung (11a, 11b, 11c 11d, 11e, 11f, 11g, 11h, 19, 20) vorhanden ist,
- dass eine in Betrieb nur achsparallel verschiebbare Eintragvorrichtung (2) vorhanden ist, um die Materialien, aus denen das Rohr aufgebaut wird, in eine oder gleichzeitig in zwei Matrizen einzubringen,
- dass eine Ausformvorrichtung vorhanden ist, um an einer ortsfesten Stelle (G) je ein fertiges Rohr aus einer Matrize herauszuziehen,
- dass eine Steuervorrichtung (29) vorhanden ist, die folgendes steuert:
= das schrittweise Drehen des Drehgestells (1),
= das Rotieren jeder Matrize (31, 32, 33, 34, 35, 36, 37, 38) mit einer von der Position, in der sie sich befindet, abhängigen Tourenzahl,
= die Verschiebebewegung der Eintragvorrichtung (2) relativ zum Drehgestell (1), sowie die momentane und die totale Menge der einzubringenden Materialien und
= die Funktionsweise der Ausformvorrichtung.

2. Anlage nach Anspruch 1, dadurch gekennzeichnet, dass alle Schleudergussmatrizen (31, 32, 33, 34, 35, 36, 37, 38) den gleichen Innendurchmesser aufweisen.

3. Anlage nach Anspruch 1, dadurch gekennzeichnet, dass mindestens eine Schleudergussmatrize einen anderen Innendurchmesser aufweist als eine der ihr benachbarten Schleudergussmatrizen.

4. Anlage nach einem der Ansprüche 1 bis 3, dadurch gekennzeichnet, dass mehrere Heizelemente (41, 42, 43, 44, 45) ortsfest angeordnet sind um die Schleudergussmatrizen zu beheizen, die sich in ihrer unmittelbaren Nähe befinden.

5. Anlage nach einem der Ansprüche 1 bis 3, dadurch gekennzeichnet, dass jede Schleudergussmatrize (31) mit einer ein- und ausschaltbaren Heizung (31c) versehen ist.

6. Anlage nach einem der Ansprüche 1 bis 5, dadurch gekennzeichnet, dass für jede Matrize (31, 32, 33, 34, 35, 36, 37, 38) an dem der Eintragvorrichtung (2) abgewandten Ende eine ortsfeste Anschluss-Öffnung (50a) der Abzugvorrichtung (50b) vorhanden ist.

7. Anlage nach einem der Ansprüche 1 bis 6, dadurch gekennzeichnet, dass die Eintragvorrichtung (2) zwei Eintragarme (57, 58) aufweist, die derart angeordnet sind, dass diese bei der achsparallelen Verschiebung der Eintragvorrichtung (2) in zwei einander benachbarte Rohre eingeführt werden.

8. Anlage nach einem der Ansprüche 1 bis 7, dadurch gekennzeichnet, dass die Steuervorrichtung (29) für jede Schleudergussmatrize (31, 32, 33, 34, 35, 36, 37, 38) einen zur Steuerung der Matrizenheizung (41, 42, 43, 44, 45; 31c) dienenden Temperaturregler enthält, um die Matrize auf eine vorprogrammierbare Temperatur aufzuheizen.

## Claims

1. Installation serving for the production of pipes consisting of filler-containing plastic, glass fibres and, if desired, further additives by the centrifugal casting method, having a rotary stand (1) which has a horizontal axis and on which several motor-drivable centrifugal moulds (31, 32, 33, 34, 35, 36, 37, 38) mounted rotatable about a horizontal axis are uniformly distributed at the same axial distance from the axis (11k) of the rotary stand, the rotary stand (1) being rotatable stepwise in such a way that, with each rotational step, a mould (32) reaches the position in which the adjacent mould (31) was previously present, wherein heating elements (41, 42, 43, 44, 45; 31c) are present for heating each mould (31, 32, 33, 34, 35, 36, 37, 38) in at least one of its positions, and wherein an extraction device (50b) is present for extracting from the individual moulds gases liberated during curing, characterized in
- that the rotary stand has six to eight centrifugal moulds (31, 32, 33, 34, 35, 36, 37, 38), each of which is provided with a drive motor (40),
- that a drive (25, 26, 27, 28) and fixing device (11a, 11b, 11c, 11d, 11e, 11f, 11g, 11h, 19, 20) is present for stepwise rotation of the rotary stand (1),
- that a feed device (2) displaceable only parallel to the axis during operation is present for introducing materials of which the pipe is composed into one mould or simultaneously into two moulds,
- that a demoulding device is present for drawing a finished pipe out of each mould at a fixed point (G),
- and that a control device (29) is present for controlling the following:
= the stepwise rotation of the rotary stand (1),
= the rotation of each mould (31, 32, 33, 34, 35, 36, 37, 38) at a speed dependent on the position in which it is located,
= the displacement movement of the feed device (2) relative to the rotary stand (1), and the instantaneous and the total amount of materials to be introduced, and
= the mode of operation of the demoulding device.

2. Installation according to Claim 1, characterized in that all centrifugal moulds (31, 32, 33, 34, 35, 36, 37, 38) have the same internal diameter.

3. Installation according to Claim 1, characterized in that at least one centrifugal mould has an internal diameter differing from that of a centrifugal mould adjacent to it.

4. Installation according to any of Claims 1 to 3, characterized in that several heating elements (41, 42, 43, 44, 45) are arranged in a stationary manner for heating the centrifugal moulds which are located in their immediate vicinity.

5. Installation according to any of Claims 1 to 3, characterized in that each centrifugal mould (31) is provided with a heater (31c) which can be switched on and off.

6. Installation according to any of Claims 1 to 5, characterized in that, for each mould (31, 32, 33, 34, 35, 36, 37, 38), a stationary connecting orifice (50a) of the demoulding device is present at the end facing away from the feed device (2).

7. Installation according to any of Claims 1 to 6, characterized in that the feed device (2) has two loading arms (57, 58) which are arranged in such a way that, when the feed device (2) is displaced parallel to the axis, they are inserted into two pipes adjacent to one another.

8. Installation according to any of Claims 1 to 7, characterized in that the control device (29) for each centrifugal mould (31, 32, 33, 34, 35, 36, 37, 38) contains a temperature controller which serves for controlling the mould heating (41, 42, 43, 44, 45; 31c), in order to heat the mould to a preprogrammable temperature.

## Revendications

1. Installation servant à fabriquer, par le procédé de coulée centrifuge, des tubes constitués de plastique contenant une charge, de fibres de verre et éventuellement d'autres additifs, comportant un bâti tournant (1) à axe horizontal sur lequel sont disposées, régulièrement réparties à une même distance de l'axe (11k) du bâti tournant, plusieurs matrices de coulée centrifuge (31, 32, 33, 34, 35, 36, 37, 38) portées avec liberté de tourner autour d'un axe horizontal et pouvant être entraînées par un moteur, dans le cas de laquelle le bâti tournant (1) peut être entraîné en rotation pas à pas de façon qu'à chaque pas de rotation une matrice (32) parvienne dans la position dans laquelle se trouvait auparavant la matrice voisine (31), dans le cas de laquelle il y a des éléments chauffants (41, 42, 43, 44, 45; 31c) pour chauffer chaque matrice (31, 32, 33, 34, 35, 36, 37, 38) dans au moins l'une de ses positions, et dans le cas de laquelle il y a un dispositif d'aspiration (50b) pour aspirer hors des différentes matrices les gaz libérés lors du durcissement, caractérisée par le fait
- que le bâti tournant présente six à huit matrices de coulée centrifuge (31, 32, 33, 34, 35, 36, 37, 38) dont chacune est équipée d'un moteur d'entraînement (40),
- que pour la rotation pas par pas du bâti tournant (1) il y a un dispositif d'entraînement (25, 26, 27, 28) et un dispositif de verrouillage (11a, 11b, 11c, 11d, 11e, 11f, 11g, 11h, 19, 20),
- qu'il y a un dispositif d'introduction (2), qui, en service, ne peut coulisser que parallèlement à l'axe, pour introduire dans une ou, simultanément, dans deux matrices les matériaux dont le tube est constitué,
- qu'il y a un dispositif de démoulage pour, en un endroit fixe (G), extraire chaque fois d'une matrice un tube fini.
- qu'il y a un dispositif de commande (29) qui commande ce qui suit:
= la rotation pas à pas du bâti tournant (1),
= la rotation de chaque matrice (31, 32, 33, 34, 35, 36, 37, 38) à une vitesse de rotation dépendant de la position dans laquelle elle se trouve,
= le mouvement de coulissement du dispositif d'introduction (2) par rapport au bâti tournant (1) ainsi que la quantité instantanée et totale des matériaux à introduire et
= le mode de fonctionnement du dispositif de démoulage.

2. Installation selon la revendication 1, caractérisée par le fait que toutes les matrices de coulée centrifuge (31, 32, 33, 34, 35, 36, 37, 38) présentent le même diamètre intérieur.

3. Installation selon la revendication 1, caractérisée par le fait qu'au moins une matrice de coulée centrifuge présente un diamètre intérieur autre que l'une des matrices de coulée centrifuge qui lui sont voisines.

4. Installation selon l'une des revendications 1 à 3, caractérisée par le fait que plusieurs éléments chauffants (41, 42, 43, 44, 45) sont disposés en un lieu fixe pour chauffer les matrices de coulée centrifuge qui se trouvent dans leur voisinage immédiat.

5. Installation selon l'une des revendications 1 à 3, caractérisée par le fait que chaque matrice de coulée centrifuge (31) est équipée d'un chauffage (31) qui peut se mettre en et hors circuit.

6. Installation selon l'une des revendications 1 à 5, caractérisée par le fait que pour chaque matrice (31, 32, 33, 34, 35, 36, 37, 38), il y a, à l'extrémité opposée au dispositif d'introduction (2), en un lieu fixe, une ouverture de raccordement (50a) du dispositif d'extraction (50b).

7. Installation selon l'une des revendications 1 à 6, caractérisée par le fait que le dispositif d'introduction (2) présente deux bras d'introduction (57, 58), qui sont disposés de façon que, lors du coulissement, parallèle à l'axe, du dispositif d'introduction (2), ces bras sont introduits dans deux tubes voisins l'un de l'autre.

8. Installation selon l'une des revendications 1 à 7, caractérisée par le fait que le dispositif de commande (29) contient, pour chaque matrice de coulée centrifuge (31, 32, 33, 34, 35, 36, 37, 38), un régulateur de température servant à commander le chauffage des matrices (41, 42, 43, 44, 45); 31c) pour porter la matrice à une température préprogrammable.
